# EUROPEAN PATENT APPLICATION

(11) **EP 4 663 124 A1**
(43) Date of publication of application: **17.12.2025**
(21) Application number: 24315292.3
(22) Date of filing: 14.06.2024
(51) Int. Cl.: A61B 5/29, A61B 5/349, A61B 5/00

(54) **TRANSFORMATION OF ACQUIRED CARDIAC SIGNALS FOR IMPROVED HEMODYNAMICS PROVIDED BY CARDIAC STIMULATION**

(71) Applicant: Sorin CRM SAS, 92140 Clamart (FR)
(72) Inventor: HERNANDEZ, Alfredo, 35510 Cesson-Sévigné (FR); FEUERSTEIN, Delphine, 92100 Boulogne Billancourt (FR); ZIGLIO, Filippo, 27100 Pavia (IT); CAZEAU, Serge, 75014 Paris (FR)
(74) Representative: Page, White & Farrer Germany LLP

(57) **Abstract**

This invention relates to an apparatus and a method for optimizing cardiac stimulation instants of a patient, wherein a signal processing chain and a set of transformations are applied to provide robust, denoised and coherent electrogram (EGM) cycles. An orthogonal (uncorrelated) transformation of observed EGMs provides an improved combination of available EGMs in terms of information content, maximizes sensitivity to changes in cardiac therapy configurations and is invariant to anatomy and lead positions of each patient. Furthermore, an original feature extraction is applied after orthogonal transformation of the EGMs and allows for calculation of a set of markers that can be better interpreted and used for optimizing the stimulation instants for a given patient.

## Description

### FIELD OF THE INVENTION

The invention relates to signal processing in cardiac therapy systems for improving cardiac stimulation.

### BACKGROUND OF THE INVENTION

The heart is a complex organ precisely controlled by the interplay of electrical and mechanical phenomena. It consists of four chambers (left and right atria (LA, RA) and left and right ventricles (LV, RV)) connected by four valves, which act in concert to regulate its filling, ejection, and overall pump function.

Among therapies that can improve the heart function in patients with heart failure, cardiac resynchronization therapy (CRT) is a minimally invasive and effective solution that improves patients' functional capacity, increases life expectancy and reduces hospitalization rate. CRT is a therapy that delivers timely electrical pulses (stimulation) to the chambers of the heart. This allows the heart to beat in a more coordinated and synchronized manner, improving the heart's pumping efficiency and increasing blood flow. Studies have shown, however, that approximately one-third of patients with advanced heart failure do not effectively respond to CRT. A major cause of poor CRT response is inappropriate atrioventricular (AV) and interventricular (VV) timing.

However, optimization of cardiac activation instants based on morphological analysis of EGM signals is particularly challenging with respect to the levels of noise observed on some EGM signals, selection of an appropriate set of EGMs to be analyzed in order to solve a particular problem, difficulties to correctly ignore redundant information and to enhance complementary information between different EGM channels, lack of appropriate observation and exploration of morphological changes (in particular for different cardiac therapy configurations), anatomy or lead placement of a particular patient and compensation of their influence on EGM morphologies, and lack of clinical interpretation of some EGM-derived markers.

### SUMMARY OF THE INVENTION

It is an object of the present invention to enhance reliability of the optimization of cardiac activation timings based on morphological analysis of EGM or other cardiac signals.

This object is achieved by a medical device as claimed in claim 1, a CRT system as claimed in claim 13, a method as claimed in claim 14, and a computer program product as claimed in claim 15.

According to first aspect, a medical device for controlling cardiac stimulation instants of a patient is provided, wherein the medical device comprises:
two or more sensors for measuring respective cardiac signals (e.g., intracardiac EGMs);
a signal processing apparatus for receiving the respective cardiac signals in the time domain and for transforming the cardiac signals into combined cardiac signals in one or more factorial planes by applying cycle segmentation and feature extraction, and for classifying an event of interest based on extracted features of the combined cardiac signals.

According to a second aspect, a method of controlling a medical device that controls cardiac stimulation instants of a patient is provided, wherein the method comprises:
measuring two or more cardiac signals (e.g., intracardiac EGMs);
receiving the respective cardiac signals in the time domain;
transforming the cardiac signals into combined cardiac signals in one or more factorial planes by applying cycle segmentation and feature extraction, and
classifying an event of interest based on extracted features of the combined cardiac signals.

According to a third aspect, a computer program product is provided, which comprises code means for producing the steps of the above method of the second aspect when run on a computer device.

According to a fourth aspect, a CRT system that comprises the medical device of the first aspect is provided.

Accordingly, the proposed signal transformation with cycle segmentation (e.g., coherent cardiac cycle determination) overcomes limitations on signal quality and ensures a stable signal morphology for further processing. The proposed representation of the electrical activation in (orthogonal) factorial planes provides a compact manner to optimally exploit complementary information, while discarding redundant information on the acquired cardiac signals.

Moreover, the proposed transformation approach can be performed on cardiac signal data derived from a database of various CRT candidates or patients (e.g., in spontaneous configuration) to provide a common representation, which is independent of lead position and anatomy of a given patient. This fact overcomes a major limitation of conventional systems where obtained classification rules may change from subject to subject.

Additionally, the proposed transformation approach can be made particularly sensitive to changes in stimulation configurations. Thereby, limitations of conventional systems concerning an appropriate choice of the set of ECG channels to be analyzed can be overcome.

Furthermore, the proposed transformation approach allows for a minimization of the number of cardiac signals to be processed, while capturing a very high degree of the observed variance.

As a result, "classic" mathematical geometrical (morphological) computations can be applied and embedded in medical devices, device program routines, and/or therapy systems to exploit features in temporal and non-temporal domains.

According to a first option of any one of the first to fourth aspects, the cycle segmentation may be performed (e.g., by the signal processing apparatus) by segmenting the received time-domain cardiac signals into cardiac cycles, selecting a specific cardiac cycle as reference cycle, determining a correlation between each cardiac cycle and the reference cycle, selecting a reference cycle that correlates with most of the cardiac cycles as dominant reference cycle, and determining a mean coherent cycle by averaging delay-corrected cardiac cycles that are correlated with the dominant reference cycle.

According to a second option which may be combined with the first option, a ratio between signal and noise may be estimated (e.g., by the signal processing apparatus) by defining all cardiac cycles that were not excluded during the cycle segmentation as the signal and by defining a result of subtracting the determined mean coherent cycle from the signal as the noise.

According to a third option which may be combined with the first or second option, one or more features of the determined mean coherent cycle may be extracted (e.g., by the signal processing apparatus) for each received cardiac signal, and one or more of the extracted features of different received cardiac signals may be combined and/or compared to characterize an overall activation of the heart for a given stimulation pattern.

According to a fourth option which may be combined with any one of the first to third options or any one of the first to fourth aspects, the cardiac signals may be transformed (e.g., by the signal processing apparatus) into the combined cardiac signals by projecting data of the cardiac signals in an orthogonal space that maximizes variance of observations, wherein each axis of the orthogonal space corresponds to another principal component derived from the cardiac signals or to another one of the cardiac signals.

According to a fifth option which may be combined with any one of the first to fourth options or any one of the first to fourth aspects, a first principal component analysis may be applied (e.g., by the signal processing apparatus), that is calculated from each of a plurality of stimulation configurations of a plurality of patients, a second principal component analysis may be applied, that is calculated from a concatenation of all stimulation configurations for each patient, a third principal component analysis may be applied, that is calculated from a selected stimulation configuration for each patient, and a fourth principal component analysis may be applied, that is calculated from a concatenation of a selected spontaneous mean coherent cycle for all patients.

According to a sixth option which may be combined with any one of the first to fifth options or any one of the first to fourth aspects, a feature analysis for feature extraction may be applied (e.g., by the signal processing apparatus) on selected principal components derived from the combined cardiac signals to obtain desired features and to use an atomic or ensemble classifier to detect the event of interest from the extracted desired features.

According to a seventh option which may be combined with the sixth option, the atomic or ensemble classifier may be configured to be subjected to a learning process via machine learning by using a learning dataset, fixed in an inference mode after a learning phase and applied in the medical device.

According to an eighth option which may be combined with any one of the first to third and fifth options, dynamic time warping may be applied (e.g., by the signal processing apparatus) on the received cardiac signals of different derivation vectors or on derived principal components to extract of indexes of similarity of morphology and/or trajectory between different configurations.

According to a ninth option which may be combined with the eighth option, an evolution of dynamic time warping similarity measures between different atrioventricular delays may be determined (e.g., by the signal processing apparatus) to provide indications about a degree of capture or fusion achieved for a certain applied atrioventricular delay.

According to a tenth option which may be combined with any one of the first to ninth options or any one of the first to fourth aspects, the two or more sensors may be configured for biventricular signal acquisition, wherein the received cardiac signals may be recorded simultaneously (e.g., by the signal processing apparatus).

According to an eleventh option which may be combined with any one of the first to tenth options or any one of the first to fourth aspects, the device may be an implantable medical device, such as a leadless pacemaker or an implantable cardioverter/defibrillator (ICD) device or a cardiac resynchronization therapy device or an implantable loop recorder (ILR) device.

It is noted that the above device may be implemented based on discrete hardware circuitries with discrete hardware components, integrated chips, or arrangements of chip modules, or based on signal processing devices or chips controlled by software routines or programs stored in memories, written on a computer readable media, or downloaded from a network, such as the Internet.

It shall be understood that the medical device of claim 1, the ICT system of claim 13, the method of claim 14, and the computer program product of claim 15 may have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a schematic representation of an implantable CRT device;
Fig. 2 shows four exemplary EGM waveforms acquired with four respective ventricular channels recorded simultaneously during three cardiac beats;
Fig. 3 shows schematically a block diagram of a signal processing apparatus or procedure for intracardiac EGMs according to a first embodiment;
Fig. 4 shows a flow diagram of a procedure for determining a mean coherent cycle for use in various embodiments;
Fig. 5 shows respective superposed waveforms of electrograms (EGMs) at different stages A to C of calculating a mean coherent cycle;
Fig. 6 shows a flow diagram of a procedure for principal component analysis for use in various embodiments;
Fig. 7 shows a diagram with projections of measured EGM channels on two principal components;
Figs. 8A and 8B show diagrams with different trajectory morphologies for different stimulation configurations in a first factorial plane spanned by two principal components for improved and non-improved patients, respectively;
Fig. 9 shows a flow diagram of a procedure for event classification for use in various embodiments;
Fig. 10 shows a schematic block diagram of a convolutional neural network for learning classifiers for event classification;
Fig. 11 shows schematically a block diagram of a signal processing apparatus or procedure for intracardiac EGMs according to a second embodiment;
Fig. 12 shows respective waveforms of dynamic time warping applied between time series;
Figs. 13A and 13B show respective waveforms of a matching derived from dynamic time warping and transformed signals according to a resulting warping path; and
Fig. 14 shows a diagram with dissimilarity measures and indexes obtained for increasing levels of AV stimulation delays.

### DETAILED DESCRIPTION OF EMBODIMENTS

Various embodiments of the present invention are now described based on a cardiac resynchronization therapy (CRT) system.

It is noted that - throughout the present disclosure - only those structural elements and functions are shown and/or described, which are useful to understand the embodiments. Other structural elements and functions are omitted for brevity reasons. Furthermore, the structure and/or function of blocks with identical reference numbers that have been described before are not described again, unless an additional specific functionality is involved.

The CRT system can be implemented as an integrated (centralized) or distributed (decentralized) system comprising at least one controller for determining and/or tracking optimal cardiac stimulation instants based on a heart sensing system which comprise a plurality of intracardiac sensing electrodes for retrieving and processing cardiac information obtained from the heart of a patient. Additionally, a medical device (e.g., a diagnostic device (such as an implantable loop recorder (ILR)) or a therapy device) that may comprise the controller may be permanently or intermittently worn (e.g., implanted or attached) by the patient, such as a heart stimulator, for applying therapeutic signals (e.g., stimulating signals). The medical device may be an implantable medical device, such as a leadless pacemaker or cardioverter/defibrillator (ICD) device, a cardiac resynchronization therapy (CRT) device, or a therapy device for assisting the heart function, e.g. of a left ventricular assist device (LVAD) pump or an artificial heart.

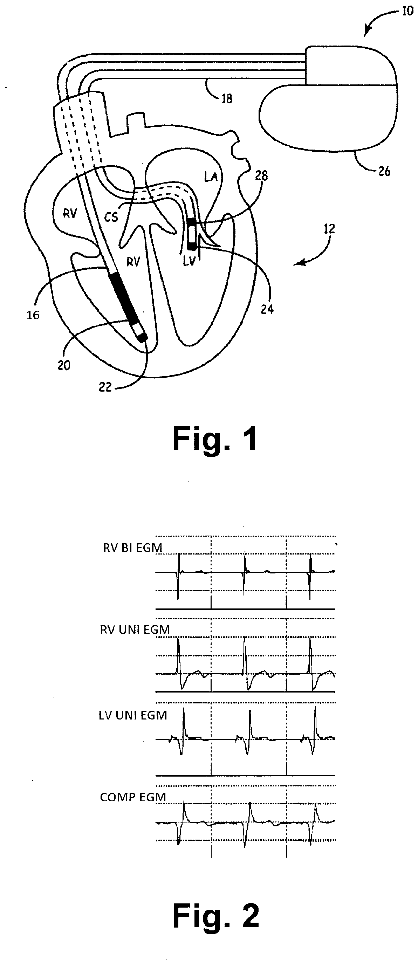

Fig. 1 shows a schematic representation of an implantable CRT device, e.g., a biventricular implantable cardioverter defibrillator (ICD).

Embodiments described herein, however, should not be interpreted as being limited to any particular implantable medical device or any particular cardiac medical device. Instead, embodiments may include any cardiac medical device so long as the device utilizes a plurality of electrodes or other sensors for monitoring the cardiac rhythm of a patient. The electrodes are capable of sensing cardiac EGM or electrocardiogram (ECG) signals, referred to herein collectively as "cardiac signals".

In Fig. 1, the right atrium (RA), left atrium (LA), right ventricle (RV), left ventricle (LV), and the coronary sinus (CS), extending from the opening in the right atrium to form the great cardiac vein, are shown schematically in heart 12. Two transvenous leads 16 and 18 connect an implantable medical device 10 with the RV and the LV, respectively. Each lead may include at least one electrical conductor and pace/sense electrode. For example, leads 16 and 18 may respectively be connected to pace/sense electrodes 20, 22, and 24, 28 which may comprise an RV ring electrode 20, an RV tip electrode 22, and two or more LV electrodes 24, 28 (which may comprise ring electrodes and optionally a tip electrode). In addition, a housing electrode 26 of the medical device 10 can be formed as part of the outer surface of the housing (e.g., can) of the medical device 10. The pace/sense electrodes 20, 22, and 24, 28 and the housing electrode 26 can be selectively employed to provide a number of unipolar (pace/sense electrode to reference potential (housing electrode 26)) and bipolar (between pace/sense electrodes) pace/sense electrode combinations for pacing and sensing functions. The depicted positions in or about the right and left heart chambers are merely illustrative. Moreover, other leads and pace/sense electrodes can be used instead of, or in combination with, any one or more of the depicted leads and electrodes.

Typically, in pacing systems of the type shown in Fig. 1, the electrodes designated herein as "pace/sense" electrodes may be used for both pacing and sensing functions. In certain embodiments, these electrodes may be used exclusively as sense electrodes in programmed or default combinations for sensing cardiac signals. The leads and electrodes described can be employed to record cardiac signals. The recorded data can be periodically transmitted to a programmer or other external device enabled for telemetric communication with the medical device 10.

The electrodes shown in Fig. 1 can be disposed in a variety of locations in the heart and are not limited to the locations shown. Furthermore, other lead and electrode systems may be substituted for the system shown in Fig. 1.

The detection, extraction and/or classification process described in embodiments herein does not require the use of electrodes for sensing atrial signals for detecting and discriminating treatable rhythms. As such, the medical device 10 is shown coupled only to ventricular leads 16 and 18 but implementation of the detection algorithm is not limited to systems employing only ventricular leads. In other embodiments, dual chamber or multi-chamber systems may be used which include atrial leads used to position electrodes in, on or around the atrial chambers.

The medical device may use ventricular EGM signals for sensing ventricular events (e.g., R-waves) for determining a need for pacing and for detecting RR intervals (i.e., the time elapsed between two successive R-waves of the QRS signal on the EGM). The cardiac stimulation optimization algorithm may employ simultaneous EGM sensing for use in estimating a heart rate, process and extract features from the raw EGMs and for applying rules to accumulate evidence to estimate the optimal cardiac stimulation intervals for a given patient.

In embodiments, at least two EGM signals may be acquired from electrodes placed at the housing 26, the RV, the LV, coils and/or the atrium, e.g., using simultaneous acquisition. A minimum of three cardiac cycles may be acquired for the same stimulation configuration (e.g., chamber stimulation, atrioventricular delay (AVD), interventricular delay (VVD), etc.).

Fig. 2 shows four exemplary EGM waveforms acquired during three heart beats with four respective ventricular channels recorded simultaneously. More specifically, the waveforms of Fig. 2 relate to an example of a biventricular (BiV) EGM signal acquisition with four ventricular EGM channels.

The uppermost waveform (RV BI EGM) of Fig. 2 corresponds to a bipolar EGM measured between tip electrode 22 and ring electrode 20 of the RV electrodes. The second-most upper waveform (RV UNI EGM) corresponds to a unipolar EGM between the RV ring electrode 20 and the housing 26 of the implanted medical device 10. The third-most upper waveform (LV UNI EGM) corresponds to unipolar EGM between one of the LV electrodes 24, 28 and the housing 26 of the medical device 10. Finally, the lowermost waveform (COMP EGM) corresponds to a composite EGM acquired between one of the LV electrodes 24, 28 and the RV ring electrode 20.

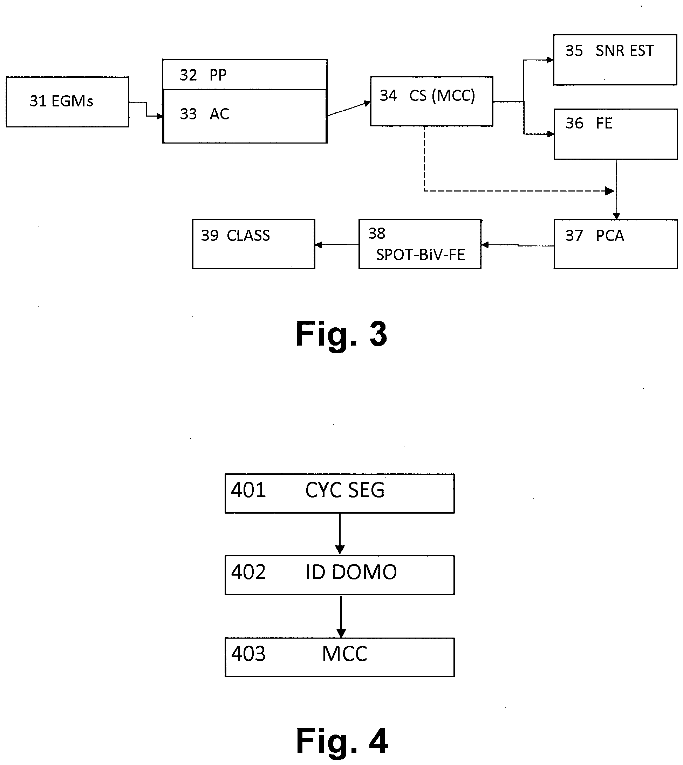

Fig. 3 shows schematically a block diagram of a signal processing apparatus or procedure for feature detection and event classification based on intracardiac EGMs according to a first embodiment, wherein raw EGM signals are transformed into optimally combined EGM signals. The signal processing apparatus may be comprised in the medical device 10 of Fig. 1 to determine optimal cardiac stimulation instants, or the signal processing procedure may be implemented as a software routine that is configured to control a processing device (e.g., digital signal processor or microcontroller) of the medical device 10 of Fig. 1 to determine optimal cardiac stimulation instants. Furthermore, the signal processing procedure (blocks/steps 32-39) may be implemented as a software routine comprised into an external computing device (computer or cloud-based system) to determine optimal cardiac stimulation instants.

The signal processing starts in block/step 31 with acquisition of multiple EGM signals (e.g., the four EGM signals of Fig. 2) as input information for optimal definition of cardiac stimulation instants.

As mentioned above, the blocks/steps of Fig. 3 may be implemented as discrete hardware circuits (e.g., application specific integrated circuit(s) (ASIC(s) or programable gate arrays (PGA(s))) or as program routines for controlling a digital signal processor (DSP) or controller or in an external computing device. In the latter case, the EGMs acquired in block/step 31 are sent wirelessly to an external system.

A pre-processing (PP) of the acquired EGMs may then be applied in block/step 32, which may comprise an artifact correction (AC) in block/step 33 for correcting (e.g., suppressing, cancelling) stimulation spikes that may lead to transient-based distortions of the EGM signals. In a possible example of digital pre-processing comprising initial sample-and-hold and analog-to-digital conversion to obtain a sequence of data samples, the artifact correction in block/step 33 may be implemented by an interpolation between data samples that precede and succeed a detected artifact (e.g., stimulation pike).

Then, in block/step 34 (CS (MCC)) cycle segmentation (e.g., peak detection and/or determination of a constant-length EGM segment representing each beat) is performed for each EGM to obtain a mean coherent cycle (e.g., mean cardiac depolarization).

In a modification of the first embodiment, a PCA is not performed during the first feature extraction part, i.e., the PCA can be performed directly at block/step 34. This is indicated by the dashed line between block/steps 34 and 37. In a particular example, the feature extraction in block/step 36 may be achieved by directly passing the original CS, without transformation.Fig. 4 shows a flow diagram of a procedure for determining the mean coherent cycle for use in various embodiments.

Determination of the mean coherent cycle for feature extractions may comprise the following three steps.

In a first step 401 (CYC SEG), cycle segmentation may be performed by segmenting the received and pre-processed time-domain EGM signals or samples (e.g., as shown in Fig. 2) into individual cardiac cycles. The segmentation may be synchronized on an atrial event, or a ventricular event, or a short duration (e.g., a few milliseconds) before or after the atrial/ventricular event. Preferably, the same synchronization should be kept for all stimulation configurations that are considered. During this process, outlier cycles are excluded, i.e., cycles that do not correspond to the configuration, such as premature ventricular contradictions (PVCs).

In subsequent step 402 (ID DOMO), a dominant morphology is identified after segmentation into cardiac cycles. In an example, the dominant morphology may be identified by taking a specific cycle (e.g., the first cycle) of the acquired sequence as reference cycle. Then, a correlation between each cycle and the reference cycles is determined (e.g., computed) for time delays (lags) in a range between a maximum negative delay (e.g., - maxLag) and a maximum positive delay (e.g., +maxLag) between a target cycle and the reference cycle. The maximum delay may range e.g. between 5 and 50ms to account for physiological variations, e.g., due to respiration or instrumental inaccuracy. Target cycles with a correlation larger than a given threshold (e.g., larger than or equal 0.8) may be considered as correlated to the reference cycle.

In examples, the correlation determination process may be repeated by taking as reference cycle the first cycle not correlated to any of the previous reference cycles. The correlation determination process may be performed separately on each of the acquired EGM signals/samples (EGM channels).

In examples, two or more iterations may be performed, unless none of the reference cycles are correlated to at least half of the cycles. In that case, the iterating process may be continued until either this condition is met, or the last cycle is reached.

At the end of the iterative process, the reference cycle that correlates with the highest number of cycles is selected as the dominant morphology. If several reference cycles are correlated with the same number of cycles, the one that led to the highest average correlation value is selected.

In subsequent step 403 (MCC), the mean coherent cycle is determined (e.g., computed) by selecting those cycles that are correlated to the dominant reference cycle, shifting these cycles according to the time delay (lag) calculated by the correlation determination process of step 402, and averaging the delay-corrected (shifted) cycles.

Note that the time delay (lag) that maximizes correlation can be calculated for each EGM channels separately. Alternatively, the time delay can be calculated on one EGM channel only and this time delay may then be applied to all other EGM channels, assuming that the EGM signals of all EGM channels are perfectly synchronized.

Similarly, correlated cycles may be selected on each EGM channel separately, or an intersection of selected cycles may be selected for all EGM channels.

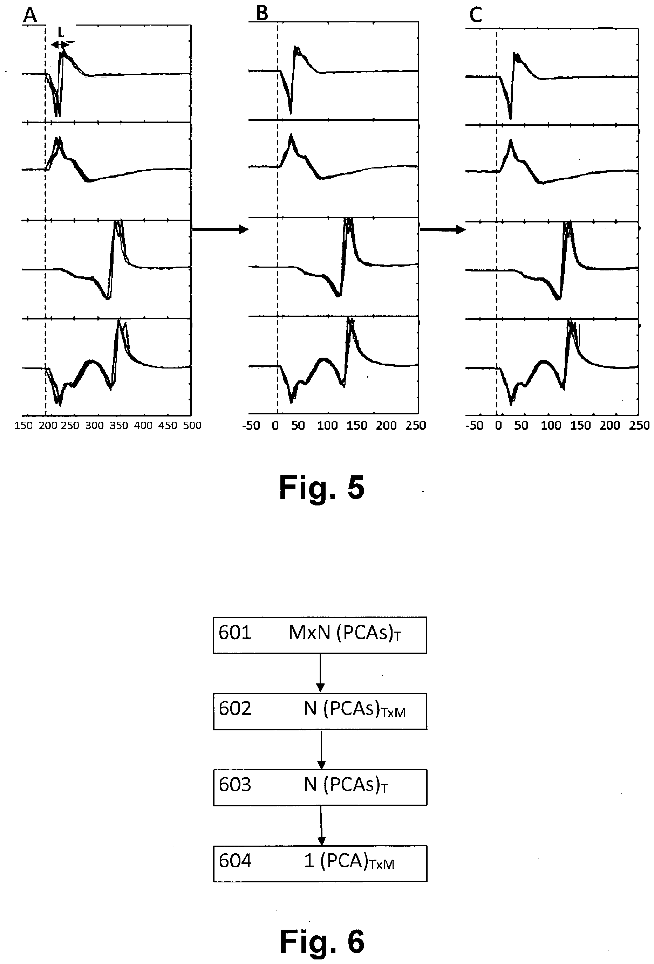

Fig. 5 shows respective waveforms of electrograms (EGMs) for the four EGM channels of Fig. 2 at different stages A to C of calculating a mean coherent cycle.

In stage A, the time delay (lag) L that maximizes correlation to the reference cycle on the RV bipolar EGM only is determined, while the horizontal time axis corresponds to the time since an atrial event. In stage B, cycle synchronization is performed using the time delay L calculated in stage A. Then, in stage C, the mean coherent cycle is determined as an ensemble average. Note that the waveforms of stage B and C correspond to the time since a ventricular event.

The resulting mean coherent cycle may be determined as the "best" representative of a given stimulation configuration at the highest signal-to-noise ratio (SNR).

In subsequent block/step 35 (SNR EST) of Fig. 3, the SNR may be estimated.

In an example, the estimation of the SNR may be calculated by defining the signal as all cycles that were not excluded during cycle segmentation and by defining the noise as the result of subtracting the determined mean coherent cycle (with appropriate time delay) from the original signal. The SNR may then be calculated as the ratio of any statistical metric(s) of the signal to the same metrics of the noise (e.g., the root mean square (RMS) of the signal).

If the SNR is below a predetermined threshold for (a) given EGM channel(s), the EGM channel may be reconfigured (e.g., using another electrode on the RV or LV lead 16, 18 of Fig. 1 for example, or another combination of electrodes) before carrying out a morphological analysis as described below. Alternatively, if some/all EGM channels show a low SNR (e.g., below the predetermined threshold) for a given stimulation configuration, the parameters (e.g., pacing vector, pacing amplitudes etc.) of the stimulation may be modified. In extreme cases, when the SNR is very low for all EGM channels in multiple stimulation configurations, the integrity of the lead itself may be questioned and further tests on the lead may need to be carried out.

In a parallel block/step 36 (FE), features (e.g., one or more of mean, slope amplitude, slope duration, first derivative, first peak, second peak, polarity, energy, piecewise integral, etc.) of the determined mean coherent cycles may be extracted for each EGM channel and may be combined/compared between different EGM channels (e.g., time differences between peaks, polarities, sum of the energy, algebraic sum of the integrals, subtractions, etc.) to characterize the overall activation of the heart for a given stimulation pattern.

A further option to extract features of multiple EGM channels may be to use time as a parameter and combine the EGM signals/samples together so as to yield an electrical activation pattern. For this pattern to be informative, a combination process may be used to maximize complementarity between the EGM channels/samples while ignoring redundancy. Furthermore, the activation pattern may need to have a spatial significance, i.e., the EGM signals/samples may need to be combined in orthogonal planes that are independent from one other (uncorrelated) and/or independent from lead position(s) and patient anatomy (contrary to the representation in Fig. 2).

The above requirements can be met by a principal component analysis (PCA) in subsequent block/step 37 where individual or coherent cycles are orthogonalized.

The principal component analysis may comprise projecting the data of the EGM channels in an orthogonal space that maximizes variance of the observations. This leads to a new coordinate system of two or more axes (coordinates) where each axis corresponds to another principal component (i.e., principal component 1 (PC1), principal component 2 (PC2), etc.) that optimally describes the variation in the data, i.e., the first coordinate (axis) may explain most of the variance, the second coordinate (axis) may explain a little less of the variance, and so on. Furthermore, these new coordinates are uncorrelated (i.e., orthogonal) to each other, meaning that complementary information between the inputs is extracted while redundant information is ignored. Each two respective orthogonal principal components form a plane with orthogonal geometrical properties and characterizations.

In an alternative embodiment, each axis of the coordinate system may directly correspond to one of the acquired EGM signals without prior principal component analysis.

Fig. 6 shows a flow diagram of a procedure for principal component analysis for use in various embodiments, which can be applied to various sets of input data, composed of M stimulation configurations and N patients for an average cycle length of T.

Steps 601-604 may be performed retrospectively, e.g., on a database content that has been acquired during a clinical study. The transformation matrixes obtained from one or more of the steps 601 to 604 may be stored and may be applied in inference mode (e.g., as a simple geometrical projection) in block/step 37 of the signal processing apparatus.

In step 601, on each of the stimulation configurations of each patient (i.e., MxN configurations), principal component analyses are performed on an input vector of size T. This results in representations that are very sensitive to the stimulation configuration but that are not comparable between one another since each representation is dependent on the lead position and stimulation parameters.

In step 602, on a concatenation of all M stimulation configurations for each patient (i.e., N concatenated configurations), principal component analyses are performed on an input vector of size TxM. The resulting principal component analyses are very useful to analyze configurations within one patient but cannot be compared to one another since their respective geometrical representation is independent of lead position and patient anatomy.

In step 603, on one stimulation configuration for each patient (i.e., N configurations), principal component analyses are performed on an input vector of size T. The selected configuration may correspond to the case where a stimulation is inhibited or where an optimal stimulation configuration has been reached (e.g., optimal being defined by an external gold-standard, such as ECG or echocardiography) or any configuration in between. This may emphasize the effect of other stimulations on the activation pattern, but the resulting representations will still be patient/lead position dependent.

In step 604, on the concatenation of the selected spontaneous (e.g., stimulation inhibition or optimum stimulation) mean coherent cycle for all patients (i.e., one concatenated configuration), a principal component analysis with input vector of size NxT is performed.

As a result, principal components can be derived from a huge variety of EGM signals acquired from different patients (e.g., based on linear combinations) to maximize variance. This has the advantage of resulting in one representation that converges to be independent of lead position (test performed) and patient anatomy. All other configurations from all patients can be projected in the new coordinate system and compared with one another. The spontaneous configuration as input of the principal component analyses is thus sensitive to CRT configuration changes that will be projected on the new coordinates.

Applying block/step 604 on a given set of CRT patients revealed that the resulting first and second principal components (PC1, PC2) alone can explain 87% of the total variance. Therefore, it can be assumed that most of the information contained by the four ventricular EGM channels of Fig. 2 can be extracted in a plane defined by these two principal components.

An exemplary projection matrix of the principal component analysis resulting from block/step 604 is presented in the below table using the designations of the EGM signals of Fig. 2, wherein first to fourth principal components are designated as PC1 to PC4:

**Table 1:**

| | PC1 | PC2 | PC3 | PC4 |
|---|---|---|---|---|
| RV BI | 0.025802 | 0.45926 | 0.88617 | -0.055863 |
| RV UNI | -0.2497 | -0.62377 | 0.28751 | -0.68257 |
| LV UNI | 0.48167 | -0.61379 | 0.33728 | 0.52677 |
| COMP | 0.83963 | 0.1525 | -0.13522 | -0.50347 |

The table 1 reveals that each principal component is a linear combination of the input EGM channels (per definition). It also gives the relative contribution/weight of each input EGM channel on the principal components. This can give insights on how to reduce the number of EGM channel inputs and therefore simplify electronics and data acquisition and processing, while collecting sufficient meaningful information for optimizing cardiac stimulation instants and/or other clinical decisions.

In embodiments, the temporal dependency/evolution of the resulting principal components may also be obtained and output (e.g., visualized) independently.

Then, in block/step 38 (SPOT-BiV-FE), a feature analysis for feature extraction (SPOT BiV feature extraction) as described in connection with the above block/step 36 may be applied on derived principal component signals.

Furthermore, since the resulting four principal components are orthogonal to each another, planes of electrical activation can be computed. Since most of the information content can be described by a combination of the first and second principal components, the first factorial plane (PC1 vs. PC2) may already provide the most significant features.

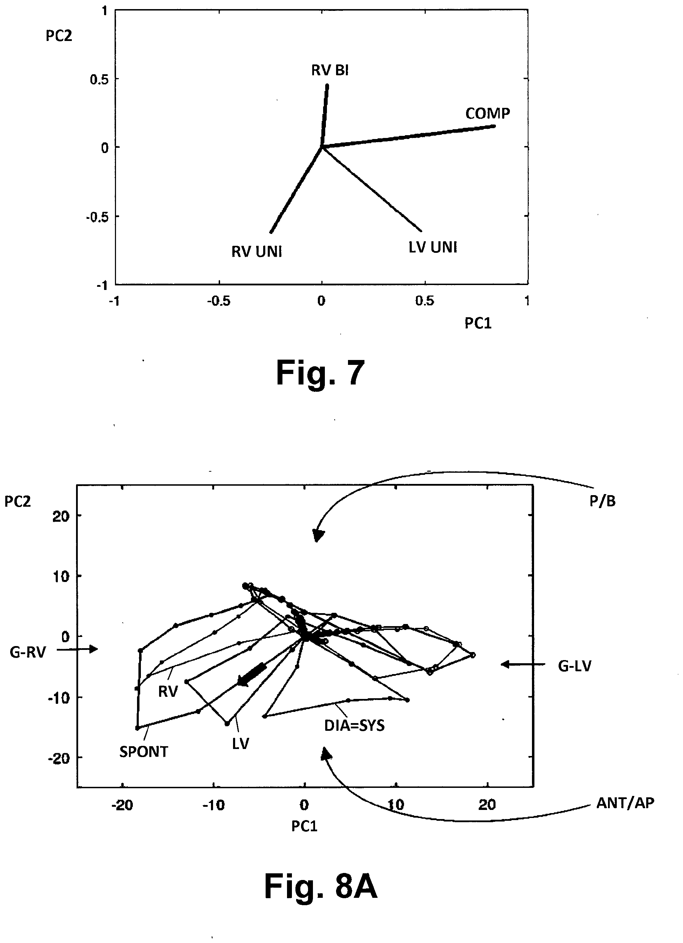

Fig. 7 shows a diagram with projections of measured EGM channels (designated as in Fig. 2) on the first two principal components PC1 and PC2. The coordinates of the two-dimensional diagram correspond to PC1 and PC2, respectively, and the projection of each of the EGM channel are shown as a respective line from the origin (0; 0) of the coordinate system to an endpoint defined by the values of PC1 and PC2 in the above table. Thus, for the bipolar RV channel (RV BI) the endpoint is located at (0.025802; 0.45926), for the unipolar RV channel (RV UNI) the endpoint is located at (-0.2497; -0.62377), for the unipolar LV channel (LV UNI) the endpoint is located at (0.48167; -0.61379), and for the composite channel (COMP) the endpoint is located at (0.83963; 0.1525).

Thus, anatomical correlations between the EGM channels can be inferred from the geometrical representation of the projection matrix in the first factorial plane spanned by PC1 and PC2. Thereby, relative importance and direction of projection of the original EGM data on a given PCA projection can be understood.

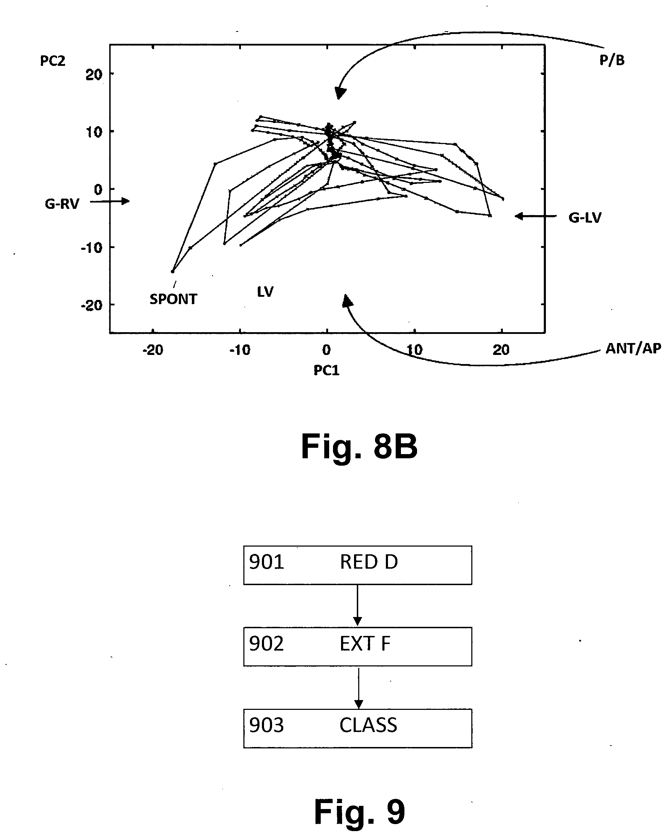

Figs. 8A and 8B show a diagrams with different trajectory morphologies for the different stimulation configurations in a first factorial plane spanned by PC1 and PC2 for improved (Fig. 8A) and non-improved (Fig. 8B) patients. Improvement may be defined here in terms of echo parameters (e.g., left pre-ejection interval (LPEI) etc.) and surface for a 12-lead ECG with standard markers (such as QRSd and axis). More specifically, the curves in the diagram of Figs. 8A and 8B indicate trajectories of different EGM configurations including spontaneous (SPONT), which is the natural rhythm of the patient, without any stimulation, left ventricle only (LV), right ventricle only (RV) and a particular stimulation configuration (DIA=SYS) that optimizes, at the same time for this particular case, ventricular filling (DIA) and the ventricular output (SYS). The thick arrow over the SPONT configuration shows the temporal direction in which these trajectories are built.

The light arrows in Fig. 8A indicate respective areas of this factorial plane, representing cardiac anatomical sites including global right ventricle (G-RV), global left ventricle (G-LV), posterior/base (P/B), and anterior/apex (ANT/AP).

Note that the diagram of Fig. 8B for the non-improved patient shows lower variations in the trajectories. Furthermore, the zone located at the center and negative part of the plane (coherent bi-ventricular activation) is only observed for the improved patient on the DIA=SYS configuration.

Thus, anatomical correlations between the EGM channels can be inferred from the resulting electrical activation patterns shown in Fig. 8 and using clinical knowledge and/or a supplementing surface ECG for example. For an optimal cardiac stimulation configuration, a compact shape of the trajectories, preferably directed towards the lower and center part of the plane is desired.

It is noted that the above-described principal component analysis in block/step 37 based on orthogonalization may be implemented based on other orthogonalization concepts using e.g. other kernels than maximization of variance of observations. For instance, an independent component analysis (ICA) may be applied, where statistical independence between observations may be used as orthogonalization criteria.

Once the optimal orthogonalization matrix has been obtained and the projection of the patient data has been applied to extract the principal components, further processing can be added in order to reduce the data to be processed, extract features from the subset of selected principal components, and/or classify the event in terms of feature values.

In block/step 39 (CLASS) of the processing of the first embodiment of Fig. 3, event classification may be applied.

Fig. 9 shows a flow diagram of a procedure for event classification for use in various embodiments.

In step 901 (RED D), data reduction can be achieved by determining how many principal components need to be used for further analysis, depending on the total amount of variance covered by each principal component. For instance, based on test results it may be verified that two principal components cover more than 80% of the variance of the total database of patient data. Thus, two principal components may be sufficient for adequate control.

In step 902 (EXT F), feature extraction may be performed to obtain desired features from the selected subset of principal components.

The desired features may comprise similarity measures (e.g., metrics) between the selected principal components based on e.g. a dynamic time warping (DTW) technique and/or on a resulting warping path, as described in the second embodiment below.

Other desired features may comprise one or more of an integral and/or energy of the selected principal components on different time supports, a histogram or occurrence map on the first or second factorial plane, a surface area, an angle, maximum/minimum values, a number of loops/lobes, a geometrical spread in the coordinate system, a difference histogram, a piecewise integral, a ratio/difference/sum of individual ones of the above desired features.

As an example of one of the possible desired features to be exploited, Figs. 7 and 8 show the first factorial planes (PC1 vs. PC2) obtained for different configurations e.g. from a database. The above-mentioned features can readily be extracted from the principal components on these two examples, providing a significant difference between most of them.

Another application example is the quantification of a level of capture or fusion for different AV delays. Morphological differences on the first factorial plane between different cycles in full capture and morphological modifications observed from fusion to capture. As an example, no morphological differences may be obtained for different atrioventricular delays (AVDs), while significant morphological differences may be obtained for different AVDs between capture and fusion. Again, the above-mentioned desired features can be extracted from the selected principal components in order to quantify this aspect.

In step 903 (CLASS), once the desired features have been extracted, an atomic or ensemble classifier can be used to detect an event of interest from the features extracted in the previous step 902. Atomic classifiers may be a set of rules, a decision tree, a neural network, etc., while ensemble classifiers may be a combination of one or many kinds and instances of atomic classifiers (e.g., random forest, etc.).

In embodiments, such classifiers may be learnt via machine learning (e.g., a neural network) from a learning dataset, fixed in an inference mode after the learning phase and applied (only in inference mode) in the device.

In the context of machine learning, a neural network is an artificial mathematical model used to approximate nonlinear functions. Neurons in an artificial neural network are usually arranged into layers, with information passing from the first layer (the input layer) through one or more intermediate layers (hidden layers) to the final layer (the output layer). The "signal" input to each neuron is a number, specifically a linear combination of the outputs of the connected neurons in the previous layer. The signal each neuron outputs is calculated from this number, according to its activation function. The behavior of the network depends on the strengths (or weights) of the connections between neurons. A network is trained by modifying these weights through empirical risk minimization or backpropagation in order to fit some preexisting dataset.

A convolutional neural network (CNN) is a regularized type of feed-forward neural network that learns feature engineering by itself via filters (or kernel) optimization. Applying cascaded convolution (or cross-correlation) kernels, less neurons are required to process tiles. Higher-layer features may be extracted from wider context windows, compared to lower-layer features.

Here, we used neural networks to classify the features of the SPOT biV. A neural network is a set of "neurons" that compute the weighted sum of the information they receive and send the output to other neurons. There are different types of neural networks that can be used, such as multilayer perceptron, residual network and shallow net, depending on the objective and on the amount of data available.

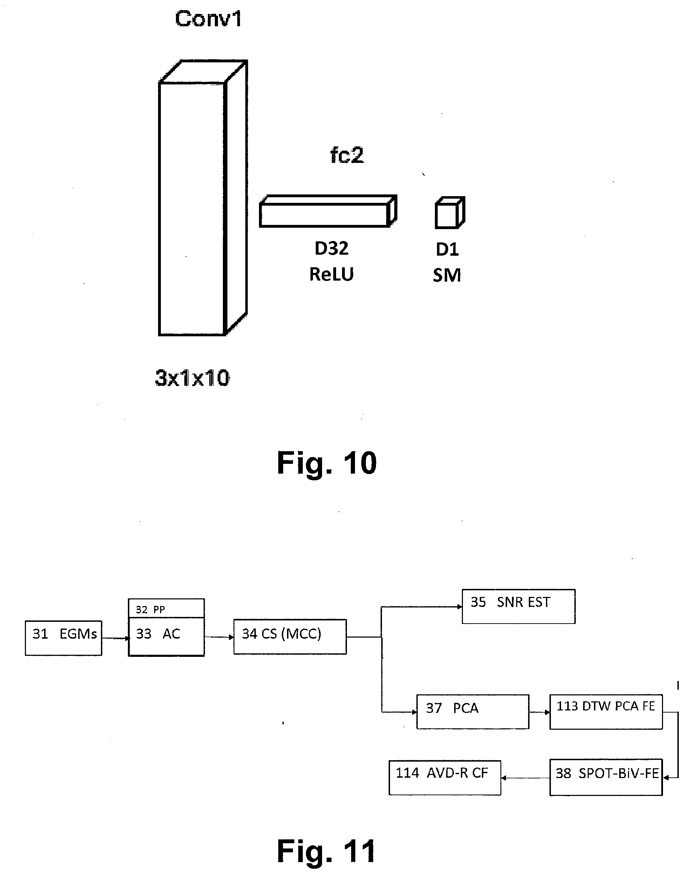

Fig. 10 shows a schematic block diagram of a shallow net configuration, which is a specific kind of CNN for learning classifiers for event classification (e.g., SPOT-BiV classification) of block/step 39 in Fig. 3 and/or step 903 in Fig. 9.

This neural network is composed of one convolution layer, a dense layer and an output layer. The convolution layer basically passes its input through one or more filters (also known as kernels). A dense layer is a layer where each neuron is connected to each neuron of the preceding layer, in a multilayer perceptron structure.

In the example of Fig. 10, the shallow net CNN architecture comprises the convolution layer (conv1) of 3x1x10 (i.e., three one-dimensional filters (since the input signals are one-dimensional signals) of order 10), a fully connected layer (fc2) of size 32 (D32 ReLU) and rectified linear activation ("ReLU") and a subsequent layer with size 1 (D1 SM) and sigmoid activation.

The ReLU activation function is a piecewise linear function that will output the input directly if it is positive, otherwise, it will output zero. It has become a default activation function for many types of neural networks because a model that uses it is easier to train and often achieves better performance.

The sigmoid activation function is used for binary classification methods with two classes only, while multiclass problems are covered by the so-called softmax actication function. Thus, the softmax function is an extension of the sigmoid function.

In an example, a database of 30 CRT patients may be used to train the CNN, wherein multiple CRT configurations are applied for each patient, i.e., BiV stimulations with various AV/VV delay combinations. In total, there may be about 900 stimulation configurations. Among these configurations, also the case of stimulation inhibition (VVI configuration) may be included.

Training inputs of the CNN could be for example:
- a concatenation of PC1 and PC2 for each configuration for each patient (i.e., 900 inputs)
- PC1 and PC2 when CRT is inhibited concatenated with PC1 and PC2 for each combination of AV/VV delay
- a bi-dimensional matrix with PC1 and PC2 when CRT is inhibited on one row, and PC1 and PC2 in a given AV/VV configuration on the second row.

Output targets for supervised learning of the CNN may be based on a response of the patient to the CRT therapy overall or a response of a given CRT configuration for each patient. The response may be defined for example as improvement, deterioration or absence of change on a gold-standard measurement, that could be assessed with echocardiography or ECG for example.

In an example, a Monte Carlo approach may be used to train the CNN, i.e., some of the patients and their CRT configurations (with their corresponding responses) may be randomly chosen in the database to train the CNN and the remaining patients and their CRT configurations are used to calculate the performances of the CNN (accuracy, loss, etc.). This process may be repeated a predefined amount of time, for example 10 times. The robustness of the CNN can be assessed using descriptive statistics (e.g., mean, standard deviation etc.) of the performances of the CNN on each of these subsets.

The process starts with the convolution layer where a set of filters (kernel) designed to detect certain features is slid over the input data (e.g., PC1/PC2 configurations of different patients, as mentioned above). The result of this process is a feature map that highlights the presence of the detected features in the signals.

The subsequent fully connected layer refers to a neural network in which each input node is connected to each output node. The fully connected layer serves as a linear combinatoric function for the nonlinear activation maps produced in the convolutional layer of the CNN. It thus combines the different activations obtain a correct event classification.

The main purpose of the sigmoid function is to transform the (unnormalised) output of the fully connected layer to a probability distribution (a normalised output), which may be represented as a vector of K elements, each of which is between 0 and 1 (i.e., a probability) and the sum of all these elements is 1 (e.g., a probability distribution). In the case of the present classification task, the ith element of the vector produced by the softmax function corresponds to the probability of the input of the network of belonging to the ith class. In an example, one class has been defined (output layer with one output), this class specifies when a given configuration for a given patient is the best ("1") or not ("0").

Other configurations were evaluated with different dimensions and orders for the convolutional layer (for instance, 3x1x25, 3x2x10, 3x2x25...); fully connected layers of dimension 32, 64, 128 and an output layer with up to three classes (output layer with up to three outputs) and different activation functions (sigmoid or softmax), each class associated with stimulation configurations leading to an improved, unchanged or deteriorated response, respectively.

Fig. 11 shows schematically a block diagram of a signal processing apparatus or procedure for intracardiac EGMs according to a second embodiment.

In the second embodiment, dynamic time warping (DTW) is used as a time series analysis for measuring similarity between two temporal sequences which may be compressed or delated with respect to each other.

In fields such as data mining and information retrieval, DTW has been successfully applied to automatically cope with time deformations and different speeds associated with time-dependent data.

DTW is a process that calculates an optimal match between two given sequences (e.g., time series) based on the following rules:
- Every index from the first sequence must be matched with one or more indices from the other sequence, and vice versa;
- the first index from the first sequence must be matched with the first index from the other sequence (but it does not have to be its only match);
- the last index from the first sequence must be matched with the last index from the other sequence (but it does not have to be its only match);
- the mapping of the indices from the first sequence to indices from the other sequence must be monotonically increasing, and vice versa.

The optimal match is the match that satisfies all the above rules and that has the minimal cost, where the cost may be computed as the sum of absolute differences, for each matched pair of indices, between their values.

The signal processing of the second embodiment differs from the first embodiment in that the feature extraction block/step 36 is replaced by a DTW-based principal component analysis feature extraction block/step (DTW PCA FE) 113. Moreover, the classification block/step 39 of the first embodiment may be replaced by an AVD range determination block/step (AVD-R CF) 114 for capture and/or fusion quantification.

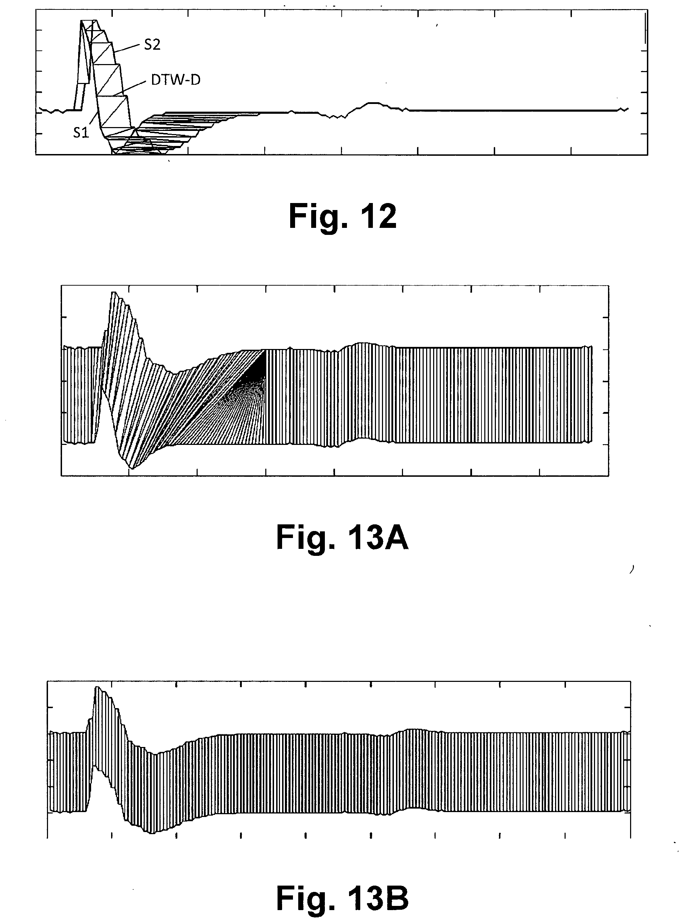

Fig. 12 shows respective waveforms of dynamic time warping applied between time series. The waveforms correspond to time series of a first signal S1 dilated with respect to a second signal S2. Furthermore, a DTW distance (DTW-D) is indicated.

In addition to a similarity measure between the two sequences, a so-called "warping path" can be produced by the DTW process. By applying a warping according to this path, the two signals S1 and S2 may be aligned in time. The signal with an original set of coordinate points X(original), Y(original) is transformed to a warped signal with coordinate points X(warped), Y(warped).

Thus, the DTW process provides similarity and transformation information.

Figs. 13A and 13B show respective waveforms of a matching derived from DTW and transformed signals according to a resulting warping path.

Fig. 13A shows matching deriving from DTW, where similar signal values are matched to each other.

Fig. 13B shows transformed signals that have been corrected according to the resulting DTW warping path.

Amerced Dynamic Time Warping (ADTW) is a variant of DTW designed to better control DTW's permissiveness in the alignments that it allows. The windows that classical DTW uses to constrain alignments introduce a step function. Any warping of the path is allowed within a predetermined window but not beyond it.

Other variants of DTW or specific algorithm constrains may be implemented to optimize the similarity measures and warping paths on the considered time series.

In block/step 113, DTW-based feature extraction is applied to the principal components derived from the principal component analysis in block/step 37.

The application of DTW on the raw EGM signals of different derivation vectors or on derived principal components allows extraction of indexes of similarity of morphology and/or trajectory between different configurations.

A possible example of application is the quantification of the level of capture or fusion for different AV delays.

Similarity measures with DTW between configurations with different AV delays and a reference configuration for example in spontaneous rhythm allows estimating a degree of closeness of myocardial electrical activation to spontaneous morphology. In contrast, similarity measures with DTW between configurations with different AV delays and the configuration in full capture reference (for example when a very short AV delay is applied), allows estimating the degree of closeness of myocardial electrical activation to the morphology in full capture condition.

Generally, the shorter the AV delay the closer the associated morphology will be to the morphology in full capture and distant from the morphology in spontaneous rhythm. As the AV delay is lengthened, on the other hand, its morphology will be more and more distant from the morphology in full capture and tending to the morphology in spontaneous rhythm.

In block/step 114 of Fig. 11, the evolution of DTW similarity measures between different AV delays and the condition in spontaneous rhythm or toward the condition in full capture or a combination of the two is determined to provide indications about the degree of capture or fusion achieved for a certain applied AV delay. The degree of capture or fusion over different AV delays can be extrapolated from the analysis of the curves of the DTW similarity/dissimilarity measures toward spontaneous and/or toward full capture through the identification of fiducial points such as change in slope, degrees of curvature, or knee points. These knee points may be the basis for the proposal of an optimal AV delay for a given patient.

It should be noted that the process of steps 113 and 114 may be implemented in the same signal processor integrating steps 31-39 of Fig. 3. In an example, the signal processor may be completely implemented into an implantable medical device. In other examples, the signal processor is implemented on an external computing system (computer or cloud) able to receive the EGM signals from the device.

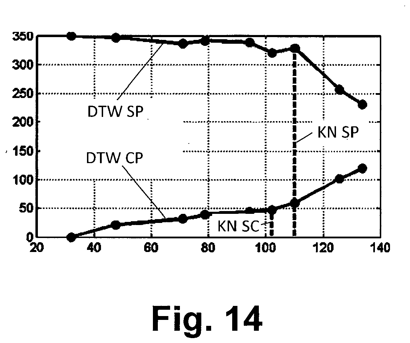

Fig. 14 shows a diagram with dissimilarity measures and indexes obtained from spontaneous and full capture references, as an example of application of DTW on principal components coming from different tested AV delays configurations. These dissimilarity measures may be calculated with respect to a spontaneous trajectory (upper series) or with respect to a full capture trajectory (lower series).

More specifically, the diagram of Fig. 14 shows DTW dissimilarity indexes (vertical axis) versus AV delay (horizontal axis) from spontaneous (DTW SP) and full capture (DTW CP) references. Additionally, knee points for spontaneous (KN SP) and full capture (KN CP) references are shown at an AVD of about 110ms and about 102ms, respectively.

The identification of knee points from the curves derived from DTW similarity measures over different AV delay configurations indicate a limit or range boundaries for capture and fusion conditions.

An optimal AV delay may be calculated as a linear or nonlinear function of these values. In an example the optimal AV delay may be automatically calculated as the min[(KN SP, KN CP)] - 20 ms. So, in this example, the suggested optimal AV delay would be 102-20 = 82 ms.

An analysis and extraction of features from the different warping paths derived from DTW can be optionally integrated as a complementary information on the identification of AV delays related to capture and fusion conditions.

To summarize, an apparatus and a method for controlling a heart rate of a patient have been described, wherein a signal processing chain and a set of transformations is applied to provide robust, denoised and coherent electrogram (EGM) cycles. An orthogonal (uncorrelated) transformation of observed EGMs provides an improved combination of available EGMs in terms of information content, maximizes sensitivity to changes in cardiac therapy configurations and is invariant to anatomy and lead positions of each patient. Furthermore, an original feature extraction is applied after orthogonal transformation of the EGMs and allows for calculation of a set of markers that can be better interpreted and used for controlling the heart rate.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The foregoing description details certain embodiments of the invention. It will be appreciated, however, that no matter how detailed the foregoing appears in the text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

The described operations or procedures like those indicated in Fig. 2 can be implemented as program code means of a computer program and/or as dedicated hardware of the receiver devices or transceiver devices, respectively. The computer program may be stored and/or distributed on a suitable medium, such as an optical storage medium or a solid-state medium, supplied together with or as part of other hardware, but may also be distributed in other forms, such as via the Internet or other wired or wireless telecommunication systems.

## Claims

1. A medical device (10) for controlling cardiac stimulation instants of a patient, comprising:
two or more sensors (20, 22, 24, 28) for measuring respective cardiac signals, in particular intracardiac electrograms, EGMs;
a signal processing apparatus (31-39; 31-35, 37, 38, 111-114) for receiving the respective cardiac signals in the time domain and for transforming the cardiac signals into combined cardiac signals in one or more factorial planes by applying feature extraction (36, 38; 38, 112, 113), and for classifying (39; 114) an event of interest based on extracted features of the featured cardiac signals.

2. The device (10) of claim 1, wherein the signal processing apparatus (31-39; 31-35, 37, 38, 111-114) is configured to perform cycle segmentation (34) by segmenting (401) the received time-domain cardiac signals into cardiac cycles, selecting a specific cardiac cycle as reference cycle, determining a correlation between each cardiac cycle and the reference cycle, selecting (402) a reference cycle that correlates with most of the cardiac cycles as dominant reference cycle, and determining (403) a mean coherent cycle by averaging delay-corrected cardiac cycles that are correlated with the dominant reference cycle.

3. The device (10) of claim 2, wherein the signal processing apparatus (31-39; 31-35, 37, 38, 111-114) is configured to estimate a ratio between signal and noise by defining all cardiac cycles that were not excluded during the cycle segmentation (34) as the signal and by defining a result of subtracting the determined mean coherent cycle from the signal as the noise.

4. The device (10) of claim 2 or 3, wherein the signal processing apparatus (31-39) is configured to extract one or more features of the determined mean coherent cycle for each received cardiac signal and to combine and/or compare the one or more extracted features of different received cardiac signals to characterize an overall activation of the heart for a given stimulation pattern.

5. The device (10) of any of the preceding claims, wherein the signal processing apparatus (31-39) is configured to transform the cardiac signals into the combined cardiac signals by projecting data of the cardiac signals in an orthogonal space that maximizes variance of observations, wherein each axis of the orthogonal space corresponds to another principal component derived from the cardiac signals or to another one of the cardiac signals.

6. The device (10) of any of the preceding claims, wherein the signal processing apparatus (31-39) is configured to apply a first principal component analysis (601) calculated from each of a plurality of stimulation configurations of a plurality of patients, to apply a second principal component analysis (602) calculated from a concatenation of all stimulation configurations for each patient, to apply a third principal component analysis (603) calculated from a selected stimulation configuration for each patient, and to apply a fourth principal component analysis (604) calculated from a concatenation of a selected spontaneous mean coherent cycle for all patients.

7. The device (10) of any of the preceding claims, wherein the signal processing apparatus (31-39; 31-35, 37, 38, 111-114) is configured to apply a feature analysis for feature extraction (38) on selected principal components derived from the combined cardiac signals to obtain desired features and to use an atomic or ensemble classifier to detect the event of interest from the extracted desired features.

8. The device (10) of claim 7, wherein the atomic or ensemble classifier is configured to be subjected to a learning process via machine learning by using a learning dataset, fixed in an inference mode after a learning phase and applied in the medical device.

9. The device (10) of claim 1 to 4 and 6, wherein the signal processing apparatus (31-35, 37, 38, 111-114) is configured to apply dynamic time warping on the received cardiac signals of different derivation vectors or on derived principal components to extract of indexes of similarity of morphology and/or trajectory between different configurations.

10. The device (10) of claim 9, wherein the signal processing apparatus (31-35, 37, 38, 111-114) is configured to determine an evolution of dynamic time warping similarity measures between different atrioventricular delays to provide indications about a degree of capture or fusion achieved for a certain applied atrioventricular delay.

11. The device (10) of any one of the preceding claims, wherein the two or more sensors (20, 22, 24, 28) are configured for biventricular signal acquisition and wherein the signal processing apparatus (31-39; 31-35, 37, 38, 111-114) is configured to record the received cardiac signals simultaneously.

12. The device of any one of the preceding claims, wherein the device is an implantable medical device (10), in particular a leadless pacemaker or an implantable cardioverter/defibrillator device or an implantable loop recorder device or a cardiac resynchronization therapy device.

13. A cardiac resynchronization therapy system comprises the medical device (10) of any one of the preceding claims.

14. A method of controlling a medical device (10) that controls cardiac stimulation instants of a patient, the method comprising:
measuring two or more cardiac signals, in particular intracardiac electrograms, EGMs;
receiving the respective cardiac signals in the time domain;
transforming the cardiac signals into combined cardiac signals in one or more factorial planes by applying feature extraction (36, 38; 38, 112, 113), and
classifying (39; 114) an event of interest based on extracted features of the combined cardiac signals.

15. A computer program product comprising code means for generating the steps of claim 14 when run on a signal processing apparatus.
